# EUROPÄISCHE PATENTANMELDUNG

(11) **EP 1 493 731 A1**
(43) Veröffentlichungstag der Anmeldung: **05.01.2005**
(21) Anmeldenummer: 04022498.2
(22) Anmeldetag: 19.03.2001
(51) Int. Cl.: C07C 211/51, C07C 215/50, C07C 217/58, C07D 295/12, C07C 215/14, C07C 233/43, C07C 215/68, A61K 7/13

(54) **N-benzyl-p-phenylendiamin-Derivate enthaltende Färbemittel für Keratinfasern sowie diese Derivate**

(30) Priorität: 31.08.2000 DE 10042787
(62) Teilanmeldung aus: 01913893.2
(71) Anmelder: Wella Aktiengesellschaft, 64274 Darmstadt (DE)
(72) Erfinder: Chassot, Laurent, Dr., 1724 Praroman (CH); Braun, Hans-Jürgen, Dr., 3182 Ueberstorf (CH)

(57) **Zusammenfassung**

N-Benzyl-p-phenylendiamin-Derivate der allgemeinen Formel (I) oder deren physiologisch verträgliche, wasserlösliche Salze, 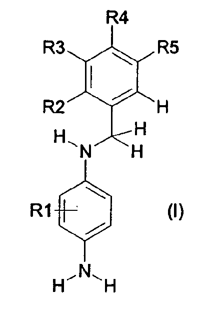 enthaltende Färbemittel für Keratinfasern sowie neue N-Benzyl-p-phenylendiamin-Derivate.

## Beschreibung

Die vorliegende Erfindung betrifft Mittel zur oxidativen Färbung von Keratinfasern, insbesondere menschlichen Haaren, auf der Basis einer Entwickler-substanz/Kupplersubstanz-Kombination, welche als Entwicklersubstanz N-Benzyl-p-phenylendiamin-Derivate enthalten, sowie neue N-Benzyl-p-phenylendiamin-Derivate.

Auf dem Gebiet der Färbung von Keratinfasern, insbesondere der Haarfärbung, haben Oxidationsfarbstoffe eine wesentliche Bedeutung erlangt. Die Färbung entsteht hierbei durch Reaktion bestimmter Entwicklersubstanzen mit bestimmten Kupplersubstanzen in Gegenwart eines geeigneten Oxidationsmittels. Als Entwicklersubstanzen werden hierbei insbesondere 2,5-Diaminotoluol, 2,5-Diaminophenylethylalkohol, p-Aminophenol und 1,4-Diaminobenzol eingesetzt, während als Kupplersubstanzen beispielsweise Resorcin, 4-Chlorresorcin, 1-Naphthol, 3-Aminophenol und Derivate des m-Phenylendiamins zu nennen sind.

An Oxidationsfarbstoffe, die zur Färbung menschlicher Haare verwendet werden, werden neben der Färbung in der gewünschten Intensität zahlreiche zusätzliche Anforderungen gestellt. So müssen die Farbstoffe in toxikologischer und dermatologischer Hinsicht unbedenklich sein und die erzielten Haarfärbungen eine gute Lichtechtheit, Dauerwellechtheit, Säureechtheit und Reibeechtheit aufweisen. Auf jeden Fall aber müssen solche Färbungen ohne Einwirkung von Licht, Reibung und chemischen Mitteln über einen Zeitraum von mindestens 4 bis 6 Wochen stabil bleiben. Außerdem ist es erforderlich, daß durch Kombination geeigneter Entwicklersubstanzen und Kupplersubstanzen eine breite Palette verschiedener Farbnuancen erzeugt werden kann.

Aus der DE-OS 34 32 214 sind bereits Mittel zum Färben von Haaren mit einem Gehalt an bestimmten N-Benzyl-p-phenylendiamin, beispielsweise N-Benzyl-p-phenylendiamin, N4-Benzyl-1,4-diamino-2-methylbenzol und 2-(((4-aminophenyl)amino)methyl)-4,6-dichlor-phenol, bekannt. Diese Verbindungen erfüllen jedoch die an Farbstoffe für Oxidationsfärbemittel gestellten Anforderungen nicht in jeder Hinsicht. Es bestand daher weiterhin ein Bedarf nach geeigneten neuen Farbstoffen.

Es wurde nun gefunden, dass bei Verwendung von N-Benzyl-pphenylendiamin-Derivaten der allgemeinen Formel (I) intensive braune, blaue und rote Farbnuancen erhalten werden.

Gegenstand der vorliegende Erfindung ist daher ein Mittel zur oxidativen Färbung von Keratinfasern, wie zum Beispiel Wolle, Pelzen, Federn oder Haaren, insbesondere menschlichen Haaren, auf der Basis einer Entwicklersubstanz-Kupplersubstanz-Kombination, das als Entwicklersubstanz mindestens ein N-Benzyl-p-phenylendiamin-Derivat der Formel (I) enthält, 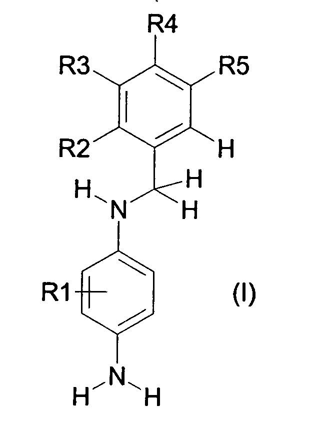 worin
- **R1**: gleich Wasserstoff, einer (C₁-C₄)-Alkylgruppe oder einer Hydroxy-(C₁-C₄)-alkylgruppe ist;
- **R2**: gleich Wasserstoff, einem Halogenatom (F, Cl, Br, J), einer Cyanogruppe, einer (C₁-C₄)-Alkoxygruppe, einer Hydroxy-(C₁-C₄)-alkoxygruppe, einer (C₁-C₆)-Alkylgruppe, einer (C₁-C₄)-Alkylthioethergruppe, einer Mercaptogruppe, einer Nitrogruppe, einer Aminogruppe, einer (C₁-C₄)-Alkylaminogruppe, einer Di-(C₁-C₄)-alkylaminogruppe, einer Di-(hydroxy-(C₁-C₄)-alkyl)aminogruppe, einer (Hydroxy-(C₁-C₄)-alkyl)aminogruppe, einer Trifluormethangruppe, einer -C(O)CH₃-Gruppe, einer -C(O)CF₃-Gruppe, einer -Si(CH₃)₃-Gruppe, einer Hydroxy-(C₁-C₄)-alkylgruppe, einer Dihydroxy-(C₃-C₄)-alkylgruppe oder eine Morpholinogruppe ist;
- **R3**: ein Halogenatom (F, Cl, Br, J), eine Hydroxygruppe, eine (C₁-C₄)-Alkoxygruppe, eine Hydroxy-(C₁-C₄)-alkoxygruppe, eine (C₁-C₆)-Alkyl-gruppe, eine (C₁-C₄)-Alkylthioethergruppe, eine Mercaptogruppe, eine Aminogruppe, eine (C₁-C₆)-Alkyl-aminogruppe, eine Di(C₁-C₆)-alkylaminogruppe, eine Di-(hydroxy-(C₁-C₄)alkyl)aminogruppe, eine Hydroxy-(C₁-C₄)-alkylaminogruppe, eine Trifluormethangruppe, eine Acetamidogruppe, eine -C(O)CH₃-Gruppe, eine -C(O)CF₃-Gruppe, eine -Si(CH₃)₃-Gruppe, eine Hydroxy-(C₁-C₄)-alkylgruppe oder eine Dihydroxy-(C₃-C₄)-alkylgruppe darstellt;
- **R4**: gleich Wasserstoff ist;
- **R5**: gleich Wasserstoff, einer Hydroxygruppe oder einer (C₁-C₆)-Alkylgruppe ist;
unter der Bedingung, dass
(i) mindestens einer der Reste **R2, R3** und **R5** von Wasserstoff verschieden ist und (ii) **R1** nicht gleich Wasserstoff oder einer (C₁-C₄)-Alkylgruppe ist, wenn gilt **R2=R5=** Wasserstoff und **R3=** Chlor.

Als Verbindungen der Formel (I) können beispielweise genannt werden: N-((2-Aminophenyl)methyl)-1,4-diaminobenzol, N-((3-Aminophenyl)-methyl)-1,4-diaminobenzol, N-((3-Hydroxyphenyl)methyl)-1,4-diaminobenzol, N-((2-(1-Hydroxyethoxy)-phenyl)-methyl)-1,4-diaminobenzol, N-((2-Methoxyphenyl)methyl)-1,4-diamino-benzol, N-((3-(1-Hydroxyethoxy)-phenyl)methyl)-1,4-diaminobenzol, N-((3-Methoxyphenyl)methyl)-1,4-diaminobenzol, N-((2-(2-Hydroxyethylamino)-phenyl)methyl)-1,4-diaminobenzol, N-((2-(Bis-(2-hydroxyethyl)amino)-phenyl)methyl)-1,4-diaminobenzol, N-((2-Dimethylamino-phenyl)methyl)-1,4-diaminobenzol, N-((2-Pyrrolidino-phenyl)methyl)-1,4-diaminobenzol, N-((3-(2-Hydroxyethylamino)-phenyl)methyl)-1,4-diaminobenzol, N-((3-(Bis-(2-hydroxyethyl)amino)-phenyl)methyl)-1,4-diaminobenzol, N-((3-Dimethylaminophenyl)methyl)-1,4-diaminobenzol, N-((3-Pyrrolidino-phenyl)methyl)-1,4-diaminobenzol, N-Benzo[1,3]dioxol-6-ylmethyl-1,4-diamino benzol, N-{2-[(4-Amino-phenylamino)-methyl]-phenyl}-acetamid, N-{3-[(4-Aminophenylamino)-methyl]-phenyl}-acetamid, N-((2,3-Diaminophenyl)methyl)-1,4-diaminobenzol, N-((2,3-Dihydroxyphenyl)methyl)-1,4-diaminobenzol, N-((2,5-Diaminophenyl)methyl)-1,4-diaminobenzol, N-((2,5-Dihydroxyphenyl)methyl)-1,4-diaminobenzol, N-((2,6-Diaminophenyl)methyl)-1,4-diaminobenzol, N-((2,6-Dihydroxy-phenyl)methyl)-1,4-diaminobenzol, N-((2-Hydroxy-3-aminophenyl)methyl)-1,4-diaminobenzol, N-((2-Hydroxy-4-aminophenyl)methyl)-1,4-diamino-benzol, N-((2-Hydroxy-5-aminophenyl)methyl)-1,4-diaminobenzol, N-((3-Hydroxy-4-aminophenyl)methyl)-1,4-diaminobenzol, N-((3-Hydroxy-5-aminophenyl)methyl)-1,4-diaminobenzol, N-((2-Amino-3-hydroxy-phenyl)methyl)-1,4-diaminobenzol, N-((2-Amino-4-hydroxyphenyl)methyl)-1,4-diaminobenzol, N¹-((2-Aminophenyl)-methyl)-2-(2-hydroxyethyl)-1,4-diaminobenzol, N¹-((2-Aminophenyl)-methyl)-2-methyl-1,4-diaminobenzol, N¹-((3-Aminophenyl)methyl)-2-(2-hydroxyethyl)-1,4-diaminobenzol, N¹-((3-Aminophenyl)methyl)-2-methyl-1,4-diaminobenzol, N¹-((3-Hydroxy-phenyl)methyl)-2-(2-hydroxyethyl)-1,4-diaminobenzol, N¹-((3-Hydroxy-phenyl)methyl)-2-methyl-1,4-diaminobenzol, N⁴-((2-Aminophenyl)methyl)-2-(2-hydroxyethyl)-1,4-diaminobenzol, N⁴-((2-Aminophenyl)methyl)-2-methyl-1,4-diaminobenzol, N⁴-((3-Aminophenyl)methyl)-2-(2-hydroxyethyl)-1,4-diaminobenzol, N⁴-((3-Aminophenyl)methyl)-2-methyl-1,4-diaminobenzol, N⁴-((3-Hydroxyphenyl)methyl)-2-(2-hydroxyethyl)-1,4-diaminobenzol, N⁴-((3-Hydroxyphenyl)methyl)-2-methyl-1,4-diaminobenzol.

Bevorzugt sind Verbindungen der Formel (I) in denen

(i) **R1** Wasserstoff bedeutet und mindestens einer der Reste **R2**, **R3** und **R5** von Wasserstoff verschieden ist, und/oder (ii) 3 der Reste **R1** bis **R5** gleich Wasserstoff sind und die beiden verbleibenden Reste unabhängig voneinander Wasserstoff, eine Methoxygruppe, eine Hydroxygruppe oder eine Aminogruppe darstellen, wobei **R2** keine Hydroxygruppe ist und mindestens einer der Reste **R2, R3** und **R5** von Wasserstoff verschieden ist; und/oder (iii) 4 der Reste **R1** bis **R5** gleich Wasserstoff sind und der 5. Rest eine Methoxygruppe, eine Hydroxyethoxygruppe, eine Hydroxygruppe oder eine Aminogruppe bedeutet, wobei **R2** keine Hydroxygruppe ist und mindestens einer der Reste **R2, R3** und **R5** von Wasserstoff verschieden ist und **R1** nicht gleich Wasserstoff oder einer (C₁-C₄)-Alkylgruppe ist, wenn gilt **R2=R5=** Wasserstoff und **R3=** Chlor.

Besonders bevorzugt sind die folgenden N-Benzyl-p-phenylendiamin-Derivate der Formel (I): N-((3-Hydroxyphenyl)methyl)-1,4-diaminobenzol und N-((2-Methoxyphenyl)methyl)-1,4-diamino-benzol sowie deren physiologisch verträglichen Salze.

Die Verbindungen der Formel (I) können sowohl als freie Basen als auch in Form ihrer physiologisch verträglichen Salze mit anorganischen oder organischen Säuren, wie zum Beispiel Salzsäure, Schwefelsäure, Phosphorsäure, Essigsäure, Propionsäure, Milchsäure oder Zitronensäure, eingesetzt werden.

Die N-Benzyl-p-phenylendiamin-Derivate der Formel (I) sind in dem erfindungsgemäßen Färbemittel in einer Gesamtmenge von etwa 0,005 bis 20 Gewichtsprozent enthalten, wobei eine Menge von etwa 0,01 bis 5 Gewichtsprozent und insbesondere 0,1 bis 2,5 Gewichtsprozent bevorzugt ist.

Als Kupplersubstanzen kommen vorzugsweise 2,6-Diamino-pyridin, 2-Amino-4-[(2-hydroxyethyl)amino]-anisol, 2,4-Diamino-1-fluor-5-methylbenzol, 2,4-Diamino-1-methoxy-5-methyl-benzol, 2,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)-amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethyl-amino-phenol, 5-Amino-2-methyl-phenol, 5-Amino-4-fluor-2-methylphenol, 5-Amino-4-methoxy-2-methyl-phenol, 5-Amino-4-ethoxy-2-methylphenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)amino]-acetamid, 5-[(2-Hydroxyethyl)amino]-2-methyl-phenol, 3-[(2-Hydroxyethyl)amino]-phenol, 3-[(2-Methoxyethyl)-amino]-phenol, 5-Amino-2-ethyl-phenol, 2-(4-Amino-2-hydroxyphenoxy)-ethanol, 5-[(3-Hydroxypropyl)amino]-2-methyl-phenol, 3-[(2,3-Dihydroxypropyl)amino]-2-methyl-phenol, 3-[(2-Hydroxyethyl)amino]-2-methylphenol, 2-Amino-3-hydroxy-pyridin, 5-Amino-4-chlor-2-methyl-phenol, 1-Naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphtholacetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diamino-benzoe-säure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol,7-Hydroxy-indol und 2,3-Indolindion in Betracht.

Obwohl die vorteilhaften Eigenschaften der hier beschriebenen Verbindungen der Formel (I) es nahelegen, diese als alleinige Entwicklersubstanz zu verwenden, ist es selbstverständlich auch möglich, diese Verbindungen gemeinsam mit bekannten Entwicklersubstanzen, wie zum Beispiel 1,4-Diaminobenzol, 2,5-Diaminotoluol, 2,5-Diaminophenylethanol, 4-Aminophenol und seinen Derivaten (beispielsweise 4-Amino-3-methylphenol), 4,5-Diamino-1-benzyl-1H-pyrazol, 4,5-Diamino-1-((4'-methylbenzyl)-1 H-pyrazol, 4,5 Diamino-1H-pyrazol, 4,5-Diamino-1-(4'-methoxybenzyl)-1 H-pyrazol, 4,5- Diamino-1-(3'-methoxy-benzyl)-1H-pyrazol, 4,5-Diamino-1-(4'-chlor-benzyl)-1H-pyrazol, 4,5-Diamino-1-((4'-methylphenyl)-1H-pyrazol, 4,5-Diamino-1-(4'-methoxy-phenyl)-1H-pyrazol, 4,5- Diamino-1-(3'-methoxy-phenyl)-1H-pyrazol, 4,5-Diamino-1-(4'-chlorphenyl)-1 H-pyrazol, 4,5-Diamino-1-(2'-hydroxyethyl)-1H-pyrazol, 4,5-Diamino-1-methyl-1H-pyrazol, 4,5-Diamino-1-ethyl-1H-pyrazol, 4-Amino-1-((4-methoxyphenyl)methyl)-5-(methylamino)-1H-pyrazol, 4-Amino-5-((2-hydroxyethyl)amino)-1-(phenylmethyl)-1H-pyrazol, 4,5-Diamino-1-methyl-3-phenyl-1H-pyrazol, 4,5-Diamino-1-(2-hydroxyethyl)-3-phenyl-1H-pyrazol, 4,5-Diamino-1,3-dimethyl-1H-pyrazol, 4,5-Diamino-3-methyl-1-phenyl-1 H-Pyrazol, 4,5-Diamino-1-(1-isopropyl)-1H-pyrazol oder Tetraaminopyrimidinen, einzusetzen.

Die Kupplersubstanzen und Entwicklersubstanzen können in dem erfindungsgemäßen Färbemittel jeweils einzeln oder im Gemisch miteinander enthalten sein, wobei die Gesamtmenge an Kupplersubstanzen und Entwicklersubstanzen in dem erfindungsgemäßen Färbemittel (bezogen auf die Gesamtmenge des Färbemittels) jeweils etwa 0,005 bis 20 Gewichtsprozent, vorzugsweise etwa 0,01 bis 5,0 Gewichtsprozent und insbesondere 0,1 bis 2,5 Gewichtsprozent, beträgt. Die Gesamtmenge der in dem hier beschriebenen Färbemittel enthaltenen Entwicklersubstanz-Kupplersubstanz-Kombination beträgt vorzugsweise etwa 0,01 bis 20 Gewichtsprozent, wobei eine Menge von etwa 0,02 bis 10 Gewichtsprozent und insbesondere 0,2 bis 6 Gewichtsprozent besonders bevorzugt ist. Die Entwicklersubstanzen und Kupplersubstanzen werden im allgemeinen in etwa äquimolaren Mengen eingesetzt; es ist jedoch nicht nachteilig, wenn die Entwicklersubstanzen diesbezüglich in einem gewissen Überschuß oder Unterschuß (beispielsweise in einem Verhältnis (Kuppler : Entwickler) von 1:2 bis 1:0,5) vorhanden sind.
Weiterhin kann das erfindungsgemäße Färbemittel zusätzlich andere Farbkomponenten, beispielsweise 6-Amino-2-methylphenol und 2-Amino-5-methylphenol, sowie ferner übliche direktziehende Farbstoffe, zum Beispiel Triphenylmethanfarbstoffe wie 4-[(4'-aminophenyl)-(4'imino-2",5"cyclohexadien-1 "-yliden)-methyl]-2-methylaminobenzol-monohydrochlorid (C.I. 42 510) und 4-[(4'amino-3'-methyl-phenyl)-(4"-imino-3"-methyl-2",5"cyclohexadien-1"-yliden)-methyl]-2-methyl-aminobenzol monohydrochlorid (C.I. 42 520), aromatische Nitrofarbstoffe wie 4-(2'-hydroxyethyl)amino-nitrotoluol, 2-Amino-4,6-dinitrophenol, 2-Amino-5-(2'-hydroxyethyl)amino-nitrobenzol, 2-Chlor-6-(ethylamino)-4-nitrophenol, 4-Chlor-N-(2-hydroxyethyl-2-nitroanilin, 5-Chlor-2-hydroxy-4-nitroanilin, 2-Amino-4-chlor-6-nitrophenol und 1-[(2'-Ureidoethyl)amino-4-nitrobenzol, Azofarbstoffe wie 6-[(4'-Aminophenyl)azo]-5-hydroxynaphthalin-1-sulfonsäure-Natriumsalz (C.I. 14 805) und Dispersionsfarbstoffe wie beispielsweise 1,4-Diaminoanthrachinon und 1,4,5,8-Tetraamino-antrachinon, enthalten. Die vorgenannten Farbkomponenten können in dem erfindungsgemäßen Färbemittel in einer Menge von etwa 0,1 bis 4 Gewichtsprozent enthalten sein.

Selbstverständlich können die Kupplersubstanzen und Entwicklersubstanzen sowie die anderen Farbkomponenten, sofern es Basen sind, auch in Form der physiologisch verträglichen Salze mit organischen oder anorganischen Säuren, wie beispielsweise Salzsäure oder Schwefelsäure, beziehungsweise - sofern sie aromatische OH-Gruppen besitzen - in Form der Salze mit Basen, zum Beispiel als Alkaliphenolate, eingesetzt werden.

Darüber hinaus können in dem erfindungsgemäßen Färbemittel, falls dieses zur Färbung von Haaren verwendet werden soll, noch weitere für kosmetische Mittel übliche Zusätze, beispielsweise Antioxidantien wie Ascorbinsäure, Thioglykolsäure oder Natriumsulfit, sowie Parfümöle, Komplexbildner, Netzmittel, Emulgatoren, Verdicker und Pflegestoffe enthalten sein.
Die Zubereitungsform des erfindungsgemäßen Färbemittels kann beispielsweise eine Lösung, insbesondere eine wässrige oder wässrigalkoholische Lösung sein. Die besonders bevorzugten Zubereitungsformen sind jedoch eine Creme, ein Gel oder eine Emulsion. Ihre Zusammensetzung stellt eine Mischung der Farbstoffkomponenten mit den für solche Zubereitungen üblichen Zusätzen dar.
Übliche Zusätze in Lösungen, Cremes, Emulsionen oder Gelen sind zum Beispiel Lösungsmittel wie Wasser, niedere aliphatische Alkohole, beispielsweise Ethanol, Propanol oder Isopropanol, Glycerin oder Glykole wie 1,2-Propylenglykol, weiterhin Netzmittel oder Emulgatoren aus den Klassen der anionischen, kationischen, amphoteren oder nichtionogenen oberflächenaktiven Substanzen wie zum Beispiel Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide und oxethylierte Fettsäureester ferner Verdicker wie hohere Fettalkohole, Stärke, Cellulosederivate, Petrolatum, Paraffinöl und Fettsäuren, sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Konzentration von etwa 0,1 bis 5 Gewichtsprozent.

Je nach Zusammensetzung kann das erfindungsgemäße Färbemittel schwach sauer, neutral oder alkalisch reagieren. Insbesondere weist es einen pH-Wert von 6,5 bis 11,5 auf, wobei die basische Einstellung vorzugsweise mit Ammoniak erfolgt. Es können aber auch organische Amine, zum Beispiel Monoethanolamin und Triethanolamin, oder auch anorganische Basen wie Natriumhydroxid und Kaliumhydroxid Verwendung finden. Für eine pH-Einstellung im sauren Bereich kommen anorganische oder organische Säuren, zum Beispiel Phosphorsäure, Essigsäure Zitronensäure oder Weinsäure, in Betracht.

Für die Anwendung zur oxidativen Färbung von Haaren vermischt man das vorstehend beschriebene Färbemittel unmittelbar vor dem Gebrauch mit einem Oxidationsmittel und trägt eine für die Haarfärbebehandlung ausreichende Menge, je nach Haarfülle, im allgemeinen etwa 50 bis 200 Gramm, dieses Gemisches auf das Haar auf. Das nach dem Vermischen mit dem Oxidationsmittel erhaltene gebrauchsfertige Oxidationshaarfärbemittel hat vorzugsweise einen pH-Wert von 6,5 bis 11,5.
Als Oxidationsmittel zur Entwicklung der Haarfärbung kommen hauptsächlich Wasserstoffperoxid oder dessen Additionsverbindungen an Harnstoff, Melamin, Natriumborat oder Natriumcarbonat in Form einer 3-bis 12-prozentigen, vorzugsweise 6-prozentigen, wässrigen Lösung, aber auch Luftsauerstoff in Betracht. Wird eine 6-prozentige Wasserstoffperoxid-Lösung als Oxidationsmittel verwendet, so beträgt das Gewichtsverhältnis zwischen Haarfärbemittel und Oxidationsmittel 5:1 bis 1:2, vorzugeweise jedoch 1:1. Größere Mengen an Oxidationsmittel werden vor allem bei höheren Farbstoffkonzentrationen im Haarfärbemittel, oder wenn gleichzeitig eine stärkere Bleichung des Haares beabsichtigt ist, verwendet. Man läßt das Gemisch bei 15 bis 50 Grad Celsius etwa 10 bis 45 Minuten lang, vorzugsweise 30 Minuten lang, auf das Haar einwirken, spült sodann das Haar mit Wasser aus und trocknet es. Gegebenenfalls wird im Anschluß an diese Spülung mit einem Shampoo gewaschen und eventuell mit einer schwachen organischen Säure, wie zum Beispiel Zitronensäure oder Weinsäure, nachgespült. Anschließend wird das Haar getrocknet.

Das erfindungsgemäße Färbemittel mit einem Gehalt an N-Benzyl-pphenylendiamin-Derivaten der Formel (I) als Entwicklersubstanz ermöglicht Färbungen mit ausgezeichneter Farbechtheit, insbesondere was die Lichtechtheit, Waschechtheit und Reibeechtheit anbetrifft. Hinsichtlich der färberischen Eigenschaften bietet das erfindungsgemäße Färbemittel je nach Art und Zusammensetzung der Farbkomponenten eine breite Palette verschiedener Farbnuancen, welche sich von blonden über braune, purpurne, violette bis hin zu blauen und schwarzen Farbtönen erstreckt. Hierbei zeichnen sich die Farbtöne durch ihre besondere Farbintensität aus. Die sehr guten färberischen Eigenschaften des Färbemittels gemäß der vorliegenden Anmeldung zeigen sich weiterhin darin, daß dieses Mittel insbesondere auch eine Anfärbung von ergrauten, chemisch nicht vorgeschädigten Haaren problemlos und mit guter Deckkraft ermöglicht.

Die Herstellung der erfindungsgemäßen N-Benzyl-p-phenylendiamin - Derivate der Formel (I) kann unter Verwendung von bekannten Syntheseverfahren, beispielsweise dem in den Ausführungsbeispielen beschriebenen Verfahren, erfolgen.

Die N-Benzyl-p-phenylendiamin-Derivate der Formel (I) sind gut in Wasser löslich und ermöglichen Färbungen mit hoher Farbintensität und ausgezeichneter Farbechtheit, insbesondere was die Lichtechtheit, Waschechtheit und Reibeechtheit anbetrifft. Sie weisen weiterhin eine ausgezeichnete Lagerstabilität, insbesondere als Bestandteil der hier beschriebenen Oxidationsfärbemitteln, auf.

Ein weiterer Gegenstand der vorliegenden Erfindung sind neue N-Benzyl-p-phenylendiamin-Derivate der allgemeinen Formel (I), wobei
(i) mindestens einer der Reste **R2, R3** und **R5** von Wasserstoff verschieden ist und (ii) **R1** nicht gleich Wasserstoff oder einer (C₁-C₄)-Alkylgruppe ist, wenn gilt **R2=R5=** Wasserstoff und **R3=** Chlor; oder deren physiologisch verträgliche, wasserlösliche Salze.

Die nachfolgenden Beispiele sollen den Gegenstand der Erfindung näher erläutern, ohne ihn darauf zu beschränken.

### Beispiele

### Beispiele 1: Synthese von N-Benzyl-1,4-diamino-benzolen

0,031 g (0,15 mmol) N-(4-Aminophenyl)-carbaminsäure-tert-butylester und 0,10 mmol des entsprechenden Aldehyds werden in 1,2-Dichlorethan gelöst. Anschliessend werden 0,1 ml einer Essigsäurelösung (1-molar in 1,2-Dichlorethan) und 0,06 g NaBH(OAc)₃ (0,3 mmol) zugegeben und die Reaktionmischung wird 5 bis 15 Stunden bei Raumtemperatur (20-25 °C) gerührt. Nach Beendigung der Reaktion wird die Reaktionsmischung in 10 ml Essigsäureethylester gegossen, die organische Phase mit Natriumhydrogencarbonat extrahiert und sodann mit Magnesiumsulfat getrocknet. Das Lösungsmittel wird am Rotationsverdampfer abdestilliert und der Rückstand an Kieselgel mit Petrolether/Essigsäureethylester (9:1) gereinigt. Das so erhaltene Produkt wird in 4 ml Ethanol und 1,5 ml einer 2,9-molaren ethanolische Salzsäurelösung auf 50°C erwärmt. Der Niederschlag wird abfiltriert, zweimal mit 1 ml Ethanol gewaschen und sodann getrocknet.

### a. N-((3-Hydroxyphenyl)methyl)-1,4-diaminobenzol Hydrochlorid

Verwendeter Aldehyd: 3-Hydroxy-benzaldehyd
Ausbeute: 0,025 g (87% der Theorie)
Massenspektrum: MH+ 215(100)

### b. N-(2-Amino-benzyl)-1,4-diamino-benzol Hydrochlorid

Verwendeter Aldehyd: 2-Amino-benzaldehyd
Ausbeute: 0,025 g (77% der Theorie)
Massenspektrum: MH+ 214(100)

### c. N-(2-Methoxy-benzyl)-1,4-diamino-benzol Hydrochlorid

Verwendeter Aldehyd: 2-Methoxy-benzaldehyd
Ausbeute: 0,025 g (83% der Theorie)
Massenspektrum: MH+ 229(100)

### d. 4-[(4-Amino-phenylamino)-methyl]-1,2-dihydroxy-benzol Hydrochlorid

Verwendeter Aldehyd: 3,4-Dihydroxy-benzaldehyd
Ausbeute: 0.025 g (82% der Theorie)
Massenspektrum: MH+ 231(100)

### e. 5-[(4-Amino-phenylamino)-methyl]-1,3-dihydroxy-benzol Hydrochlorid

Verwendeter Aldehyd: 3,5-Dihydroxy-benzaldehyd
Ausbeute: 0,025 g (82% der Theorie)
Massenspektrum: MH+ 231(100)

### f. 5-(4-Amino-phenyl)aminomethyl-1,3-diamino-benzol Hydrochlorid

Verwendeter Aldehyd: 3,5-Diamino-benzaldehyd
Ausbeute: 0,025 g (66% der Theorie)
Massenspektrum: MH+ 228(100)

### g. N-(2-Morpholin-4-yl-benzyl)-1,4-diamino-benzol Hydrochlorid

Verwendeter Aldehyd: 2-Morpholino-benzaldehyd
Ausbeute: 0,025 g (70% der Theorie)

### Beispiele 2: Synthese von N¹- Benzyl-1,4-Diamino-2-methyl-benzolen und N⁴-Benzyl-1,4-Diamino-2-methyl-benzolen

0,033 g (0,15 mmol) eine Mischung aus N-(4-Amino-2-methyl-phenyl)-carbaminsäure-tert-butylester und von N-(4-Amino-3-methyl-phenyl)-carbaminsäure-tert-butylester und 0,1 mmol des entsprechenden Aldehyds werden in 1,2-Dichlorethan gelöst. Anschliessend werden 0,1 ml einer Essigsäurelösung (1-molar in 1,2-Dichlorethan) und 0,06 g NaBH(OAc)₃ (0,3 mmol) zugegeben und die Reaktionmischung wird 5 bis 15 Stunden bei Raumtemperatur (20-25 °C) gerührt. Nach Beendigung der Reaktion wird die Reaktionsmischung in 10 ml Essigsäureethylester gegossen, die organische Phase mit Natriumhydrogencarbonat extrahiert und sodann mit Magnesiumsulfat getrocknet. Das Lösungsmittel wird am Rotationsverdampfer abdestilliert und der Rückstand an Kieselgel mit Petrolether/Essigsäureethylester (9:1) gereinigt. Das so erhaltene Produkt wird in 4 ml Ethanol und 1,5 ml einer 2,9-molaren ethanolische Salzsäurelösung auf 50 °C erwärmt. Der Niederschlag wird abfiltriert, zweimal mit 1 ml Ethanol gewaschen und sodann getrocknet.

### a. 4-Amino-2-[(4-amino-2-methyl-phenylamino)-methyl]-phenol Hydrochlorid und 4-Amino-2-[(4-amino-3-methyl-phenylamino)-methyl]-phenol Hydrochlorid

Verwendeter Aldehyd: N-(4-Hydroxy-3-formyl-phenyl)-carbaminsäure-tertbutylester
Ausbeute: 0,025 g (35% der Theorie)
Massenspektrum: MH+ 244(100)

### b. N¹-(2-Methoxy-benzyl)-2-methyl-1,4-diamino-benzol Hydrochlorid und N¹-(2-Methoxy-benzyl)-3-methyl-1,4-diamino-benzol Hydrochlorid

Verwendeter Aldehyd: 2-Methoxy-benzaldehyd
Ausbeute: 0,025 g (39% der Theorie)
Massenspektrum: MH+ 243(100)

### c. N¹-(3-Amino-benzyl)-2-methyl-1,4-diamino-benzol Hydrochlorid und N¹-(3-Amino-benzyl)-3-methyl-1,4-diamino-benzol Hydrochlorid

Verwendeter Aldehyd: 3-Amino-benzaldehyd
Ausbeute: 0,025 g (37% der Theorie)
Massenspektrum: MH+ 228(100)

### d. 3-[(4-Amino-2-methyl-phenylamino)-methyl]-phenol Hydrochlorid und 3-[(4-Amino-3-methyl-phenylamino)-methyl]-phenol Hydrochlorid

Verwendeter Aldehyd: 3-Hydroxybenzaldehyd
Ausbeute: 0,025 g (41% der Theorie)
Massenspektrum: MH+ 229(100)

### e. 5-(4-Amino-2-methyl-phenyl)aminomethyl-1,3-diamino-benzol Hydrochlorid un 5-(4-Amino-3-methyl-phenyl)aminomethyl-1,3-diamino-benzol Hydrochlorid

Verwendeter Aldehyd: 3,5-Diamino-benzaldehyd
Ausbeute: 0,025 g (32% der Theorie)
Massenspektrum: MH+ 243(100)

### f. 2-{4-[(4-Amino-2-methyl-phenylamino)-methyl]-phenoxy}-ethanol Hydrochlorid und 2-{4-[(4-Amino-3-methyl-phenylamino)-methyl]-phenoxy}-ethanol Hydrochlorid

Verwendeter Aldehyd: 4-(2-Hydroxy-ethoxy)-benzaldehyd
Ausbeute: 0,025 g (36% der Theorie)
Massenspektrum: MH+ 273(100)

### g. 2-[{4-[(4-Amino-2-methyl-phenylamino)-methyl]-phenyl}-(2-hydroxyethyl)-amino]-ethanol und 2-[{4-[(4-Amino-3-methyl-phenylamino)-methyl]-phenyl}-(2-hydroxy-ethyl)-amino]-ethanol

Verwendeter Aldehyd: 4-(Bis-(2-hydroxyethyl)-amino)-benzaldehyd
Ausbeute: 10 g (16% der Theorie)

### h. N¹-(2-Amino-benzyl)-2-methyl-1,4-diamino-benzol Hydrochlorid und N¹-(2-Amino-benzyl)-3-methyl-1,4-diamino-benzol Hydrochlorid

Verwendeter Aldehyd: 2-Amino-benzaldehyd
Ausbeute: 0,025 g (37% der Theorie)

### i. 2-{4-[(4-Amino-2-methyl-phenylamino)-methyl]-phenoxy}-ethanol Hydrochlorid und 2-{4-[(4-Amino-3-methyl-phenylamino)-methyl]-phenoxy}-ethanol Hydrochlorid

Verwendeter Aldehyd: 4-(2-Hydroxy-ethoxy)-benzaldehyd
Ausbeute: 0,025 g (36% der Theorie)
Massenspektrum: MH+ 273(100)

### j. 2-Methyl-N¹-(2-morpholin-4-yl-benzyl)-1,4-diamino-benzol Hydrochlorid und 3-Methyl-N¹-(2-morpholin-4-yl-benzyl)-1,4-diamino-benzol Hydrochlorid

Verwendeter Aldehyd: 2-Morpholino-benzaldehyd
Ausbeute: 0,025 g (30% der Theorie)

### Beispiele 3: Synthese von N¹-Benzyl-1,4-diamino-2-(2-hydroxyethyl)-benzolen und N⁴-Benzyl-1,4-diamino-2-(2-hydroxyethyl)-benzolen

0,038 g (0,15 mmol) eine Mischung von N-(4-Amino-2-(2-hydroxyethyl)-phenyl)-carbaminsäure-tert-butylester und von N-(4-Amino-3-(2-hydroxyethyl)-phenyl)-carbaminsäure-tert-butylester und 0,1 mmol des entsprechenden Aldehyds werden in 1,2-Dichlorethan gelöst. Anschliessend werden 0,1 ml einer Essigsäurelösung (1-molar in 1,2-Dichlorethan) und 0,06 g NaBH(OAc)₃ (0,3 mmol) zugegeben und die Reaktionmischung wird 5 bis 15 Stunden bei Raumtemperatur (20-25 °C) gerührt. Nach Beendigung der Reaktion wird die Reaktionsmischung in 10 ml Essigsäureethylester gegossen, die organische Phase mit Natriumhydrogencarbonat extrahiert und sodann mit Magnesiumsulfat getrocknet. Das Lösungsmittel wird am Rotationsverdampfer abdestilliert und der Rückstand an Kieselgel mit Petrolether/Essigsäureethylester (9:1) gereinigt. Das so erhaltene Produkt wird in 4 ml Ethanol und 1,5 ml einer 2,9 molaren ethanolische Salzsäurelösung auf 50°C erwärmt.

Der Niederschlag wird abfiltriert, zweimal mit 1 ml Ethanol gewaschen und sodann getrocknet.

### a. 2-[5-Amino-2-(3-amino-benzylamino)-phenyl]-ethanol Hydrochlorid und 2-[6-Amino-3-(3-amino-benzylamino)-phenyl]-ethanol Hydrochlorid

Verwendeter Aldehyd: 3-Amino-benzaldehyd
Ausbeute: 0,025 g (34% der Theorie)
Massenspektrum: MH+ 258(100)

### b. 2-[5-Amino-2-(4-amino-benzylamino)-phenyl]-ethanol Hydrochlorid und 2-[6-Amino-3-(4-amino-benzylamino)-phenyl]-ethanol Hydrochlorid

Verwendeter Aldehyd: N-(4-Formyl-phenyl)-carbaminsäure-tert.butylester
Ausbeute: 0,025 g (34% der Theorie)
Massenspektrum: MH+ 258(50)

### c. 2-[5-Amino-2-(2-methoxy-benzylamino)-phenyl]-ethanol Hydrochlorid und 2-[6-Amino-3-(2-methoxy-benzylamino)-phenyl]-ethanol Hydrochlorid

Verwendeter Aldehyd: 2-methoxy-benzaldehyd
Ausbeute: 0,025 g (36% der Theorie)
Massenspektrum: MH+ 273(100)

### d. 2-[5-Amino-2-(2-amino-benzylamino)-phenyl]-ethanol Hydrochlorid und 2-[6-Amino-3-(2-amino-benzylamino)-phenyl]-ethanol Hydrochlorid

Verwendeter Aldehyd: 2-Amino-benzaldehyd
Ausbeute: 0,025 g (34% der Theorie)
Massenspektrum: MH+ 258(100)

### e. 2-{[4-Amino-2-(2-hydroxy-ethyl)-phenylamino]-methyl}-1,4-dihydroxybenzol Hydrochlorid und 2-{[4-Amino-3-(2-hydroxy-ethyl)-phenylamino]-methyl}-1,4-dihydroxy-benzol Hydrochlorid

Verwendeter Aldehyd: 3,6-Dihydroxy-benzaldehyd
Ausbeute: 0,025 g (36% der Theorie)
Massenspektrum: MH+ 275(100)

### f. 4-Amino-2-{[4-amino-2-(2-hydroxy-ethyl)-phenylamino]-methyl}-phenol Hydrochlorid und 4-Amino-2-{[4-amino-3-(2-hydroxy-ethyl)-phenylamino]-methyl}-phenol Hydrochlorid

Verwendeter Aldehyd: N-(4-Hydroxy-3-formyl-phenyl)-carbaminsäure-tertbutylester
Ausbeute: 0,025 g (32% der Theorie)
Massenspektrum: MH+ 274(100)

### g. 2-[5-Amino-2-(2-morpholin-4-yl-benzylamino)-phenyl]-ethanol Hydrochlorid und 2-[6-Amino-3-(2-morpholin-4-yl-benzylamino)-phenyl]-ethanol Hydrochlorid

Verwendeter Aldehyd: 2-Morpholino-benzaldehyd
Ausbeute: 0,025 g (28% der Theorie)

### h. 2-[2 -Amino-5-(3,5-diamino-benzylamino)-phenyl]-ethanol Hydrochlorid und 2-[5-Amino-2-(3,5-diamino-benzylamino)-phenyl]-ethanol Hydrochlorid

Verwendeter Aldehyd: 3,5-Diamino-benzaldehyd
Ausbeute: 0,025 g (29% der Theorie)
Massenspektrum: MH+ 273(100)

### Beispiele 4 bis 28: Haarfärbemittel

Es werden Haarfärbelösungen der folgenden Zusammensetzung hergestellt:

| | |
|---|---|
| 1,25 mmol | Entwicklersubstanz der Formel (I) gemäß Tabelle 1 |
| 1,25 mmol | Kupplersubstanz gemäß Tabelle 1 |
| 1,0 g | Kaliumoleat (8-prozentige wässrige Lösung) |
| 1,0 g | Ammoniak (22-prozentige wässrige Lösung) |
| 1,0 g | Ethanol |
| 0,3 g | Ascorbinsäure |
| ad 100,0 g | Wasser |

50 g der vorstehenden Färbelösung werden unmittelbar vor der Anwendung mit 50 g einer 6-prozentigen wässrigen Wasserstoffperoxidlösung vermischt. Anschliessend wird das Gemisch auf gebleichte Haare aufgetragen. Nach einer Einwirkungszeit von 30 Minuten bei 40 °C wird das Haar mit Wasser gespült, mit einem handelsüblichen Shampoo gewaschen und getrocknet. Die resultierenden Färbungen sind in Tabelle 1 zusammengefaßt.

**Tabelle 1:**

| **Beispiel Nr.** | **Ent- wickler- substanz der Formel (I)** | **Kupplersubstanz** | | | |
|---|---|---|---|---|---|
| | | **I. 1,3-Di-hydroxy-benzol** | **II. 1,3-Diamino-4-(2-hydroxy-ethoxy)-benzol-sulfat** | **III. 5-Amino-2-methyl-phenol** | **IV. 1-Naphtol** |
| **4.** | gemäß | braun | dunkelblau | purpur | blau |
| | Beispiel **1a** | | | | |
| **5.** | gemäß | mittel- | blau | purpur | blau |
| | Beispiel **1b** | blond | | | |
| **6.** | gemäß | hell- | blau | purpur | blaugrau |
| | Beispiel **1c** | blond | | | |
| **7.** | gemäß | blond | blau | purpur | blaugrau |
| | Beispiel **1d** | | | | |
| **8.** | gemäß | dunkel- | blau | purpur | blaugrau |
| | Beispiel **1e** | blond | | | |
| **9.** | gemäß | braun | blau | purpurblau | blaugrau |
| | Beispiel **1f** | | | | |
| **10.** | gemäß | hell- | blau | purpur | hellviolett |
| | Beispiel **1g** | blond | | | |
| **11.** | gemäß | blond | blau | purpur | violett |
| | Beispiel **2a** | | | | |
| **12.** | gemäß | mittel- | blau | purpur | violett |
| | Beispiel **2b** | blond | | | |
| **13.** | gemäß | mittel- | blau | purpur | blau |
| | Beispiel **2c** | blond | | | |
| **14.** | gemäß | mittel- | blau | purpur | violett |
| | Beispiel **2d** | blond | | | |
| **15.** | gemäß | blond | blau | purpur | violett |
| | Beispiel **2e** | | | | |
| **16.** | gemäß | hell- | blau | purpur | violett |
| | Beispiel **2f** | blond | | | |
| **17.** | gemäß | hell- | blau | purpur | violett |
| | Beispiel **2g** | blond | | | |
| **18.** | gemäß | hell- | blau | purpur | violett |
| | Beispiel **2h** | blond | | | |
| **19.** | gemäß | hell- | blau | purpur | violett |
| | Beispiel **2i** | blond | | | |
| **20.** | gemäß Beispiel **2j** | hell-blond | blau | purpur | violett |
| **21.** | gemäß Beispiel **3a** | hell-blond | blau | purpur | blau |
| **22.** | gemäß Beispiel **3b** | dunkel-blond | blau | purpur | violett |
| **23.** | gemäß Beispiel **3c** | hell-blond | blau | purpur | violett |
| **24.** | gemäß Beispiel **3d** | hell-blond | blau | purpur | hellblau |
| **25.** | gemäß Beispiel **3e** | hell-blond | blau | purpur | violett |
| **26.** | gemäß Beispiel **3f** | hell-blond | blau | purpur | violett |
| **27.** | gemäß Beispiel **3g** | hell-blond | blau | purpur | hellblau |
| **28.** | gemäß Beispiel **3h** | hell-blond | blau | purpur | violett |

### Beispiele 29 bis 38: Haarfärbemittel

Es werden Haarfärbelösungen der folgenden Zusammensetzung hergestellt:

| | |
|---|---|
| X g | N-(Benzyl)-1,4-diamino-benzol (Entwicklersubstanz |
| | **E1** der Formel (I) gemäß Tabelle 2) |
| U g | Entwicklersubstanz **E8** bis **E15** gemäß Tabelle 2 |
| Y g | Kupplersubstanz **K11** bis **K36** gemäß Tabelle 4 |
| Z g | direktziehender Farbstoff **D1** bis **D3** gemäß Tabelle 3 |
| 10,0 g | Kaliumoleat (8-prozentige wässrige Lösung) |
| 10,0 g | Ammoniak (22-prozentige wässrige Lösung) |
| 10,0 g | Ethanol |
| 0,3 g | Ascorbinsäure |
| ad 100,0 g | Wasser |

30 g der vorstehenden Färbelösung werden unmittelbar vor der Anwendung mit 30 g einer 6prozentigen wässsrigen Wasserstoffperoxidlösung vermischt. Anschliessend wird das Gemisch auf gebleichte Haare aufgetragen. Nach einer Einwirkungszeit von 30 Minuten bei 40°C wird das Haar mit Wasser gespült, mit einem handelsüblichen Shampoo gewaschen und getrocknet. Die Färbeergebnisse sind in Tabelle 5 zusammengefasst.

### Beispiele 39 bis 44: Haarfärbemittel

Es werden cremeförmige Farbträgermassen der folgenden Zusammensetzung hergestellt:

| | |
|---|---|
| X g | N-(Benzyl)-1,4-Diamino-benzol (Entwicklersubstanz **E1** |
| | der Formel (I) gemäß Tabelle 2) |
| U g | Entwicklersubstanz **E8** bis **E15** gemäß Tabelle 2 |
| Y g | Kupplersubstanz **K11** bis **K36** gemäss Tabelle 4 |
| Z g | direktziehender Farbstoff **D2** gemäss Tabelle 3 |
| 15,0 g | Cetylalkohol |
| 0,3 g | Ascorbinsäure |
| 3,5 g | Natriumlaurylalkoholdiglycolethersulfat, 28-prozentige |
| | wässrige Lösung |
| 3,0 g | Ammoniak, 22-prozentige wässrige Lösung |
| 0,3 g | Natriumsulfit, wasserfrei |
| ad 100 g | Wasser |

30 g der vorstehenden Färbecreme werden unmittelbar vor der Anwendung mit 30 g einer 6-prozentigen Wasserstoffperoxidlösung vermischt. Anschliessend wird das Gemisch auf das Haar aufgetragen. Nach einer Einwirkzeit von 30 Minuten bei 40°C wird das Haar mit Wasser gespült, mit einem handelsüblichen Shampoo gewaschen und getrocknet. Die Färbeergebnisse sind in Tabelle 6 zusammengefasst.

**Tabelle 2:**

| **Entwicklersubstanzen** | |
|---|---|
| **E1** | N-((3-Hydroxyphenyl)methyl)-1,4-diaminobenzol Hydrochlorid |
| | |
| **E8** | 1,4-Diaminobenzol |
| **E9** | 2,5-Diamino-phenylethanol-sulfat |
| **E10** | 3-Methyl-4-amino-phenol |
| **E11** | 4-Amino-2-aminomethyl-phenol-dihydrochlorid |
| **E12** | 4-Amino-phenol |
| **E13** | N,N-Bis(2'-hydroxyethyl)-p-phenylendiamin-sulfat |
| **E14** | 4,5-Diamino-1-(2'-hydroxyethyl)-pyrazol-sulfat |
| **E15** | 2,5-Diaminotoluol-sulfat |

**Tabelle 3:**

| **Direktziehende Farbstoffe** | |
|---|---|
| **D1** | 2,6-Diamino-3-((pyridin-3-yl)azo)pyridin |
| **D2** | 6-Chlor-2-ethylamino-4-nitro-phenol |
| **D3** | 2-Amino-6-chlor-4-nitro-phenol |

**Tabelle 4:**

| **Kupplersubstanzen** | |
|---|---|
| **K11** | 1,3-Diaminobenzol |
| **K12** | 2-Amino-4-(2'-hydroxyethyl)amino-anisol-sulfat |
| **K13** | 1,3-Diamino-4-(2'-hydroxyethoxy)benzol-sulfat |
| **K14** | 2,4-Diamino-5-fluor-toluol-sulfat |
| **K15** | 3-Amino-2-methylamino-6-methoxy-pyridin |
| **K16** | 3,5-Diamino-2,6-dimethoxy-pyridin-dihydrochlorid |
| **K17** | 2,4-Diamino-5-ethoxy-toluol-sulfat |
| **K18** | N-(3-Dimethylamino)phenylharnstoff |
| **K19** | 1,3-Bis(2,4-Diaminophenoxy)propan-tetrahydrochlorid |
| **K21** | 3-Amino-phenol |
| **K22** | 5-Amino-2-methyl-phenol |
| **K23** | 3-Amino-2-chlor-6-methyl-phenol |
| **K24** | 5-Amino-4-fluor-2-methyl-phenol-sulfat |
| **K25** | 1-Naphthol |
| **K26** | 1-Acetoxy-2-methyl-naphthalin |
| **K31** | 1,3-Dihydroxy-benzol |
| **K32** | 2-Methyl-1,3-dihydroxy-benzol |
| **K33** | 1-Chlor-2,4-dihydroxy-benzol |
| **K34** | 4-(2'-Hydroxyethyl)amino-1,2-methylendioxybenzol-hydrochlorid |
| **K35** | 3,4-Methylendioxy-phenol |
| **K36** | 2-Amino-5-methyl-phenol |

**Tabelle 5:**

| Haarfärbemittel | | | | |
|---|---|---|---|---|
| **Beispiel Nr.** | **29** | **30** | **31** | **32** |
| **Farbstoffe** | (Farbstoffmenge in Gramm) | | | |
| **E1** | 0,25 | 0,20 | 0,20 | 0,20 |
| | | | | |
| **E10** | 0,30 | | | |
| **E11** | | 0,30 | | |
| **E12** | | | 0,30 | |
| **E14** | | | | 0,30 |
| | | | | |
| **K31** | 0,18 | | | 0,20 |
| **K32** | | 0,22 | | |
| **K33** | | | 0,20 | |
| **K25** | 0,30 | 0,30 | | 0,30 |
| **K26** | | | 0,35 | |
| **Färbeergebnis** | rotbraun | rotbraun | rotbraun | rotbraun |

**Tabelle 5**

| (Fortsetzung) | | | | | | |
|---|---|---|---|---|---|---|
| **Beispiel Nr.** | **33** | **34** | **35** | **36** | **37** | **38** |
| **Farbstoffe** | (Farbstoffmenge in Gramm) | | | | | |
| **E1** | 0,35 | 0,25 | 0,3 | 0,10 | 0,10 | 0,15 |
| | | | | | | |
| **E8** | | | | 0,15 | | |
| **E9** | | | | | 0,15 | |
| **E15** | | | | | | 0,15 |
| | | | | | | |
| **K12** | | | 0,10 | | | |
| **K13** | 0,09 | 0,09 | | | | |
| **K31** | 0,20 | | | 0,15 | 0,20 | 0,10 |
| **K32** | | 0,20 | | 0,10 | | 0,10 |
| **K33** | | | 0,20 | | | |
| **K21** | 0,05 | | | | | |
| **K22** | | 0,05 | | | | |
| **K23** | | | 0,05 | 0,10 | 0,10 | 0,10 |
| **Färbeergebnis** | blond | blond | blond | blond | blond | blond |

**Tabelle 6:**

| Haarfärbemittel | | | | | | |
|---|---|---|---|---|---|---|
| **Beispiel Nr.** | **39** | **40** | **41** | **42** | **43** | **44** |
| **Farbstoffe** | (Farbstoffmenge in Gramm) | | | | | |
| **E1** | 1,80 | 1,80 | 1,80 | 0,70 | 0,70 | 0,70 |
| | | | | | | |
| **K12** | | | | 0,10 | 0,10 | 0,10 |
| **K13** | 1,10 | 1,10 | 1,10 | | | |
| **K31** | 1,10 | 1,10 | 1,10 | 0,40 | 0,40 | 0,40 |
| | | | | | | |
| **D2** | | | | 0,10 | 0,10 | 0,10 |
| **K23** | | | 0,05 | 0,10 | 0,10 | 0,10 |
| **Färbeergebnis** | schwarz | schwarz | schwarz | braun | braun | braun |

Alle in der vorliegenden Anmeldung enthaltenen Prozentangaben stellen soweit nicht anders angegeben Gewichtsprozente dar.

## Patentansprüche

1. N-Benzyl-p-phenylendiamin-Derivate der allgemeinen Formel (I) oder deren physiologisch verträgliche, wasserlösliche Salze, 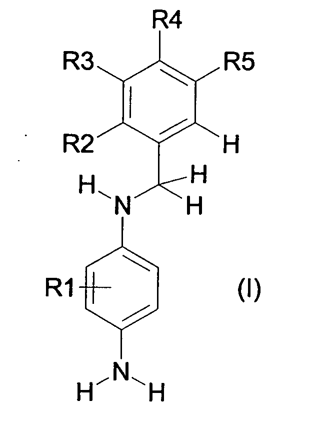 worin
**R1** gleich Wasserstoff, einer (C₁-C₄)-Alkylgruppe oder einer Hydroxy-(C₁-C₄)-alkylgruppe ist ;
**R2** gleich Wasserstoff, einem Halogenatom, einer Cyanogruppe, einer (C₁-C₄)-Alkoxygruppe, einer Hydroxy-(C₁-C₄)-alkoxygruppe, einer (C₁-C₆)-Alkylgruppe, einer (C₁-C₄)-Alkylthioethergruppe, einer Mercaptogruppe, einer Nitrogruppe, einer Aminogruppe, einer (C₁-C₄)-Alkylaminogruppe, einer Di-(C₁-C₄)-alkylaminogruppe, einer Di-(hydroxy-(C₁-C₄)-alkyl)aminogruppe, einer (Hydroxy-(C₁-C₄)-alkyl)-aminogruppe, einer Trifluormethangruppe, einer -C(O)CH₃-Gruppe, einer -C(O)CF₃-Gruppe, einer -Si(CH₃)₃-Gruppe, einer Hydroxy-(C₁-C₄)-alkylgruppe, einer Dihydroxy-(C₃-C₄)-alkylgruppe oder eine Morpholinogruppe ist;
**R3** Wasserstoff, ein Halogenatom, eine Hydroxygruppe, eine (C₁-C₄)-Alkoxygruppe, eine Hydroxy-(C₁-C₄)-alkoxygruppe, eine (C₁-C₆)-Alkylgruppe, eine (C₁-C₄)-Alkylthioethergruppe, eine Mercaptogruppe, eine Aminogruppe, eine (C₁-C₆)-Alkylaminogruppe, eine Di-(C₁-C₆)-alkylaminogruppe, eine Di-(hydroxy-(C₁-C₄)-alkyl)aminogruppe, eine Hydroxy-(C₁-C₄)-alkylaminogruppe, eine Trifluormethangruppe, eine Acetamidogruppe, eine -C(O)CH₃-Gruppe, eine -C(O)CF₃-Gruppe, eine -Si(CH₃)₃-Gruppe, eine Hydroxy-(C₁-C₄)-alkylgruppe oder eine Dihydroxy-(C₃-C₄)-alkylgruppe darstellt;
**R4** gleich Wasserstoff ist;
**R5** gleich Wasserstoff, einer Hydroxygruppe oder einer (C₁-C₆)-Alkylgruppe ist; unter der Bedingung, dass
(i) mindestens einer der Reste **R2, R3** und **R5** von Wasserstoff verschieden ist und (ii) **R1** nicht gleich Wasserstoff oder einer (C₁-C₄)-Alkylgruppe ist, wenn gilt **R2=R5=** Wasserstoff und **R3=** Chlor.

2. Verbindung der Formel (I) gemäß Anspruch 1, **dadurch gekennzeichnet, dass** (i) **R1** Wasserstoff bedeutet und mindestens einer der Reste **R2, R3** und **R5** von Wasserstoff verschieden ist, und/oder (ii) 3 der Reste **R1** bis **R5** gleich Wasserstoff sind und die beiden verbleibenden Reste unabhängig voneinander Wasserstoff, eine Methoxygruppe, eine Hydroxygruppe oder eine Aminogruppe darstellen, wobei **R2** keine Hydroxygruppe ist und mindestens einer der Reste **R2, R3** und **R5** von Wasserstoff verschieden ist; und/oder (iii) 4 der Reste **R1** bis **R5** gleich Wasserstoff sind und der 5. Rest eine Methoxygruppe, eine Hydroxyethoxygruppe, eine Hydroxygruppe oder eine Aminogruppe bedeutet, wobei R2 keine Hydroxygruppe ist und mindestens einer der Reste **R2, R3** und **R5** von Wasserstoff verschieden ist; **R1** nicht gleich Wasserstoff oder einer (C₁-C₄)-Alkylgruppe ist, wenn gilt **R2=R5=** Wasserstoff und **R3=** Chlor.

3. Mittel zur oxidativen Färbung von Keratinfasern auf der Basis einer Entwicklersubstanz-Kupplersubstanz-Kombination, **dadurch gekennzeichnet, dass** es als Entwicklersubstanz mindestens ein N-Benzyl-p-phenylendiamin-Derivat der allgemeinen Formel (I) oder dessen physiologisch verträgliche, wasserlösliche Salze enthält, 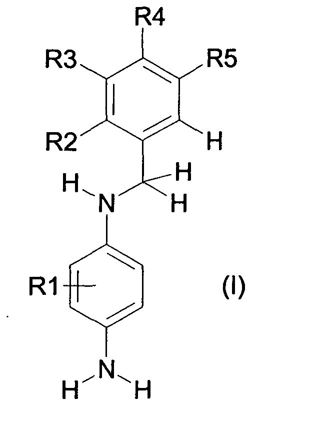 worin
**R1** gleich Wasserstoff, einer (C₁-C₄)-Alkylgruppe oder einer Hydroxy-(C₁-C₄)-alkylgruppe ist;
**R2** gleich Wasserstoff, einem Halogenatom (F, Cl, Br, J), einer Cyanogruppe, einer (C₁-C₄)-Alkoxygruppe, einer Hydroxy-(C₁-C₄)-alkoxygruppe, einer (C₁-C₆)-Alkylgruppe, einer (C₁-C₄)-Alkylthioethergruppe, einer Mercaptogruppe, einer Nitrogruppe, einer Aminogruppe, einer (C₁-C₄)-Alkylaminogruppe, einer Di-(C₁-C₄)-alkylaminogruppe, einer Di-(hydroxy-(C₁-C₄)-alkyl)aminogruppe, einer (Hydroxy-(C₁-C₄)-alkyl)-aminogruppe, einer Trifluormethangruppe, einer -C(O)CH₃-Gruppe, einer -C(O)CF₃-Gruppe, einer -Si(CH₃)₃-Gruppe, einer Hydroxy-(C₁-C₄)-alkylgruppe, einer Dihydroxy-(C₃-C₄)-alkylgruppe oder eine Morpholinogruppe ist;
**R3** Wasserstoff, ein Halogenatom, eine Hydroxygruppe, eine (C₁-C₄)-Alkoxygruppe, eine Hydroxy-(C₁-C₄)-alkoxy-gruppe, eine (C₁-C₆)-Alkylgruppe, eine (C₁-C₄)-Alkylthioethergruppe, eine Mercaptogruppe, eine Aminogruppe, eine (C₁-C₆)-Alkylaminogruppe, eine Di-(C₁-C₆)-alkylaminogruppe, eine Di-(hydroxy-(C₁-C₄)-alkyl)aminogruppe, eine Hydroxy-(C₁-C₄)-alkylaminogruppe, eine Trifluormethangruppe, eine Acetamidogruppe, eine -C(O)CH₃-Gruppe, eine -C(O)CF₃-Gruppe, eine -Si(CH₃)₃-Gruppe, eine Hydroxy-(C₁-C₄)-alkylgruppe oder eine Dihydroxy-(C₃-C₄)-alkylgruppe darstellt;
**R4** gleich Wasserstoff ist;
**R5** gleich Wasserstoff, einer Hydroxygruppe oder einer (C₁-C₆)-Alkylgruppe ist; unter der Bedingung, dass (i) mindestens einer der Reste **R2, R3** und **R5** von Wasserstoff verschieden ist und (ii) **R1** nicht gleich Wasserstoff oder einer (C₁-C₄)-Alkylgruppe ist, wenn gilt **R2=R5=** Wasserstoff und **R3=** Chlor.

4. Mittel nach Anspruch 3, **dadurch gekennzeichnet, dass** (i) **R1** Wasserstoff bedeutet, wobei mindestens einer der Reste **R2, R3** und **R5** von Wasserstoff verschieden ist; und/oder (ii) 3 der Reste **R1** bis **R5** gleich Wasserstoff sind und die beiden verbleibenden Reste unabhängig voneinander Wasserstoff, eine Methoxygruppe, eine Hydroxygruppe oder eine Aminogruppe darstellen, wobei **R2** keine Hydroxygruppe ist und mindestens einer der Reste **R2, R3** und **R5** von Wasserstoff verschieden ist; und/oder (iii) 4 der Reste **R1** bis **R5** gleich Wasserstoff sind und der 5. Rest eine Methoxygruppe, eine Hydroxyethoxygruppe, eine Hydroxygruppe oder eine Aminogruppe bedeutet, wobei **R2** keine Hydroxygruppe ist und mindestens einer der Reste **R2, R3** und **R5** von Wasserstoff verschieden ist.

5. Mittel nach Anspruch 3 oder 4, **dadurch gekennzeichnet, dass** die Verbindung der Formel (I) ausgewählt ist aus der Gruppe bestehend aus N-((3-Hydroxyphenyl)methyl)-1,4-diaminobenzol und N-((2-Methoxyphenyl)methyl)-1,4-diamino-benzol sowie den physiologisch verträglichen Salzen dieser Verbindungen.

6. Mittel nach einem der Ansprüche 3 bis 5, **dadurch gekennzeichnet, dass** das N-Benzyl-p-phenylendiamin-Derivat der Formel (I) in einer Menge von 0,005 bis 20 Gewichtsprozent enthalten ist.

7. Mittel nach einem der Ansprüche 3 bis 6, **dadurch gekennzeichnet, dass** es einen pH-Wert von 6,5 bis 11,5 aufweist.

8. Mittel nach einem der Ansprüche 3 bis 7, **dadurch gekennzeichnet, dass** die Kupplersubstanz ausgewählt ist aus der Gruppe bestehend aus 2,6-Diamino-pyridin, 2-Amino-4-[(2-hydroxyethyl)amino]-anisol, 2,4-Diamino-1-fluor-5-methyl-benzol, 2,4-Diamino-1-methoxy-5-methylbenzol, 2,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)-amino]-anilin, 1,3-Di(2,4-diaminophenoxy)-propan, Di(2,4-diamino-phenoxy)-methan, 1,3-Diamino-2,4-dimethoxy-benzol, 2,6-Bis(2-hydroxy-ethyl)amino-toluol, 4-Hydroxyindol, 3-Dimethylamino-phenol, 3-Diethyl-amino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlorphenol, 3-Amino-2-methyl-phenol, 3-Amino-2-chlor-6-methyl-phenol, 3-Amino-phenol, 2-[(3-Hydroxyphenyl)amino]-acetamid, 5-[(2-Hydroxyethyl)amino]-2-methyl-phenol, 3-[(2-Hydroxyethyl)amino]-phenol, 3-[(2-Methoxyethyl)-amino]-phenol, 5-Amino-2-ethyl-phenol, 2-(4-Amino-2-hydroxyphenoxy)-ethanol, 5-[(3-Hydroxypropyl)amino]-2-methyl-phenol, 3-[(2,3-Dihydroxypropyl)amino]-2-methyl-phenol, 3-[(2-Hydroxyethyl)-amino]-2-methyl-phenol, 2-Amino-3-hydroxy-pyridin, 5-Amino-4-chlor-2-methyl-phenol, 1-Naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxy-phenol, 3,4-Methylendioxy-anilin, 5-[(2-Hydroxyethyl)-amino]-1,3-benzodioxol, 6-Brom-1-hydroxy-3,4-methylendioxy-benzol, 3,4-Diamino-benzoe-säure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxyindol,7-Hydroxy-indol und 2,3-Indolindion.

9. Mittel nach einem der Ansprüche 3 bis 8, **dadurch gekennzeichnet, dass** die Entwicklersubstanzen und Kupplersubstanzen, bezogen auf die Gesamtmenge des Färbemittels, jeweils in einer Gesamtmenge von 0,005 bis 20 Gewichtsprozent enthalten sind.

10. Mittel nach einem der Ansprüche 3 bis 9, **dadurch gekennzeichnet, dass** es zusätzlich mindestens einen direktziehenden Farbstoff enthält.

11. Mittel nach einem der Ansprüche 3 bis 10, **dadurch gekennzeichnet, dass** es ein Haarfärbemittel ist.
